# EUROPEAN PATENT SPECIFICATION

(11) **EP 2 004 540 B1**
(45) Date of publication and mention of the grant of the patent: **20.05.2015**
(21) Application number: 07753641.5
(22) Date of filing: 21.03.2007
(51) Int. Cl.: A61C 5/06, B01F 15/04, B01F 5/06

(54) **SELF-CONTAINED SINGLE DOSE DUAL FLUID DISPENSER**
IN SICH GESCHLOSSENER EINZELDOSENSPENDER FÜR ZWEI FLÜSSIGKEITEN
DISPOSITIF AUTONOME D'ADMINISTRATION DE DEUX FLUIDES À DOSE UNIQUE

(30) Priority: 31.03.2006 US 788144 P
(43) Date of publication of application: 24.12.2008
(73) Proprietor: NORDSON CORPORATION, Westlake, OH 44145-1119 (US)
(72) Inventor: BRENNAN, Robert C., Bordentown, NJ 08505 (US); METZBOWER, Curt, Washington Crossing, PA 18977 (US); PAPPALARDO, Matthew E., Leonia, NJ 07605 (US)
(74) Representative: Eisenführ Speiser
(86) International application number: PCT/US2007/007031
(87) International publication number: WO 2007/126656

(56) References cited:
- WO-A-2007/126532
- US-A- 3 570 713
- US-A- 5 310 091
- US-A- 5 566 860
- US-A1- 2006 144 858
- US-A1- 2006 151 530
- US-B1- 6 520 381
- US-B2- 6 817 988

## Description

### CROSS-REFERENCE TO RELATED APPLICATIONS

This application claims priority from and claims the benefit of U.S. Provisional Application No. 60/788,144, filed March 31, 2006, entitled "Self-Contained Single Dose Dual Fluid Dispenser", which is hereby incorporated by reference

### BACKGROUND

In certain fields or applications, there is a need for dual fluid chemically reactive components (i.e. a resin and a hardener) that can be dispensed in a single dose. The dental field is an example of such a field. In the dental field, dental practitioners prefer using dispensers that can be disposed of after use with one patient. These are typically referred to as single dose dispensers. Single dose dispensers provide several significant advantages over multi-use dispensers. Single dose dispensers are more sanitary than multi-use dispensers. With a single use dispenser, the dental practitioner can dispose of the dispenser after the procedure is complete; thereby, reducing the possibility of spreading germs and infection among patients. Also, single dose dispensers do not have as much waste as multi-use dispensers. With a multi-use dispenser, the resin and hardener components often times cross-contaminate between uses, causing the adhesive to harden and rendering the dispenser useless. The dental practitioner, as a result in this case, is only able to get two to three uses out of the multi-use dispenser and does not achieve the benefit of using the dispenser multiple times. Also, single dose dispensers, because they are smaller, are much easier to work with and manipulate, especially in the confined space of a patient's mouth.

As such, most dental practitioners prefer single dose dispensers which can be disposed of after use with a single patient. The problem for practitioners today is that to make a single dose for a two component end product, they either have to hand mix or batch mix the components together. Both of these methods have drawbacks. Hand mixing is usually done by dispensing the two components separately from tubes and then hand mixing the components together to make the end product. This process is time consuming and cumbersome and not the most efficient way for a dental practitioner to mix a two component end product. The other way practitioners mix a single dose of a two component end product is to batch mix the two components that need to be mixed together. An example of such a single dose mixing system is the GuttaFlow® mixing system. In the GuttaFlow® mixing system, the container contains gutta-percha in particle form and a sealer. When the dental practitioner is ready to use the end product, the practitioner breaks the seal separating the gutta-percha from the sealer and mixes the entire contents of the two components together in a standard triturator. Upon completion of mixing, a single batch of the end product is made and can be dispensed for use. In this system, the components of the end product are all mixed together at one time and are not mixed together only as needed as they are dispensed from the cartridge.

Accordingly, there is a need for a single dose, two component self-contained dispenser that mixes the two components of the end product together as the two components are dispensed from the dispenser.

### SUMMARY

According to one aspect of the present invention, a self-contained single dose dual fluid dispenser to store and dispense two fluids includes a dual fluid container having an outer cartridge wall defining an outlet and an open end opposite the outlet. The dual fluid dispenser also includes a delivery tube disposed within the outer cartridge wall and defines an outlet that is co-located with the outlet defined by the outer cartridge wall; a first piston disposed between the outer cartridge wall and the delivery tube which forms a fluid chamber for a single dose of a first fluid and a second piston disposed within the outer cartridge wall between the first piston and the open end of the outer cartridge wall. The dual fluid dispenser further includes a fixed wall disposed between the first piston and the second piston, wherein the fixed wall and the second piston define a fluid chamber for a single dose of a second fluid. A force providing mechanism is disposed within the open end of and attached to the dual fluid container, wherein, when activated, the force providing mechanism applies a force to the second piston. Also included is a transmission structure disposed between the second piston and the first piston.

### DESCRIPTION OF THE DRAWINGS

These and other features, aspects and advantages of the present invention will become better understood with regard to the following description, appended claims and accompanying drawings where:
FIG. 1A is a perspective view of a syringe embodiment of a dispenser of the present invention with a cap in place;
FIG. 1B is a side view of the syringe embodiment of a dispenser of the present invention with a mixing element attached;
FIG. 1C is a longitudinal sectional view taken along the line 1C-1C of Fig. 1B, illustrating a filled embodiment of a dual fluid dispenser of the present invention;
FIG. 1D is a longitudinal sectional view of the dual fluid dispenser depicted in FIG. 1C in an intermediate dispensing position;
FIG. 1E is a longitudinal sectional view of the dual fluid dispenser depicted in FIG. 1C with the contents of the dual fluid cartridge dispensed; and
FIGS. 2A-2E are a set of figures similar to FIGS. 1A-1E for a screw advance mechanism embodiment of the dual fluid dispenser which does not fall within the scope of the present invention;
FIGS. 3A-3E are a set of figures similar to FIGS. 1A-1E for a spiral advance mechanism embodiment of the dual fluid dispenser which does not fall within the scope of the present invention; and
FIGS. 4A-4E are a set of figures similar to FIGS. 1A-1E for a potential energy advance mechanism embodiment of the dual fluid dispenser which does not fall within the scope of the present invention.

### DETAILED DESCRIPTION

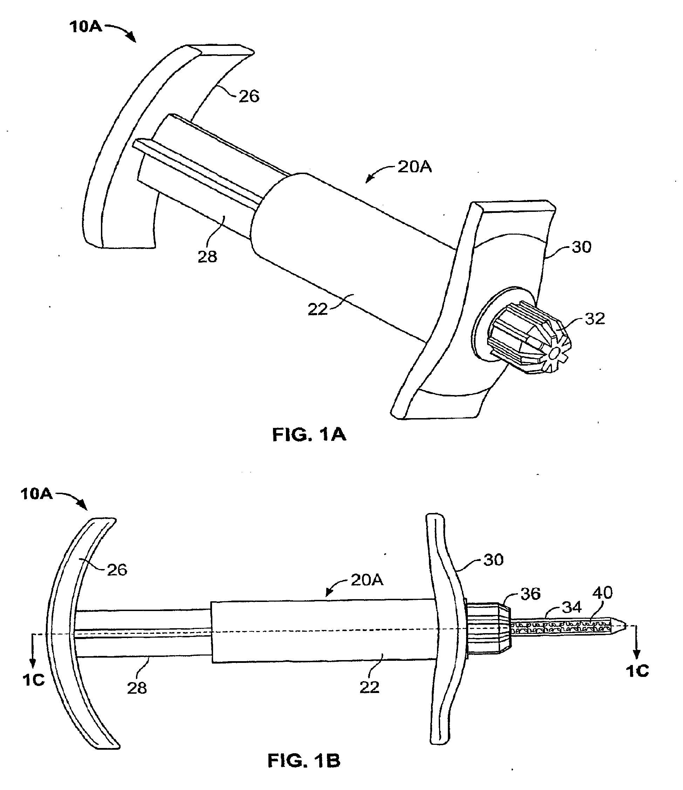

Figs. 1A-1E, 2A-2E, 3A-3E and 4A-4E illustrate four different embodiments of self-contained, single dose, dual fluid dispensers 10A-10D . The embodiments depicted herein are 1:1 fluid ratio embodiments of the dual fluid dispensers 10A-10D, but it should be understood that other embodiments of the dispensers with other fluid ratios (e.g. 2:1 ratio) could be utilized without departing from the invention disclosed herein. Also, the dispensers disclosed herein may be used in any suitable field of use or application.

Referring specifically to the embodiment depicted in Figs. 1A-1E, a self-contained, single dose syringe embodiment 10A is depicted. The syringe 10A includes a dual fluid container 20A, a handle 26 with a plunger 28 and a grip 30 at the end of the dual fluid container 20A opposite the handle 26. As explained in detail below, pressing the handle 26, in conjunction with grasping the grip 30, generates a force, which is used to dispense the fluids contained in the dual fluid container 20A from the syringe 10A.

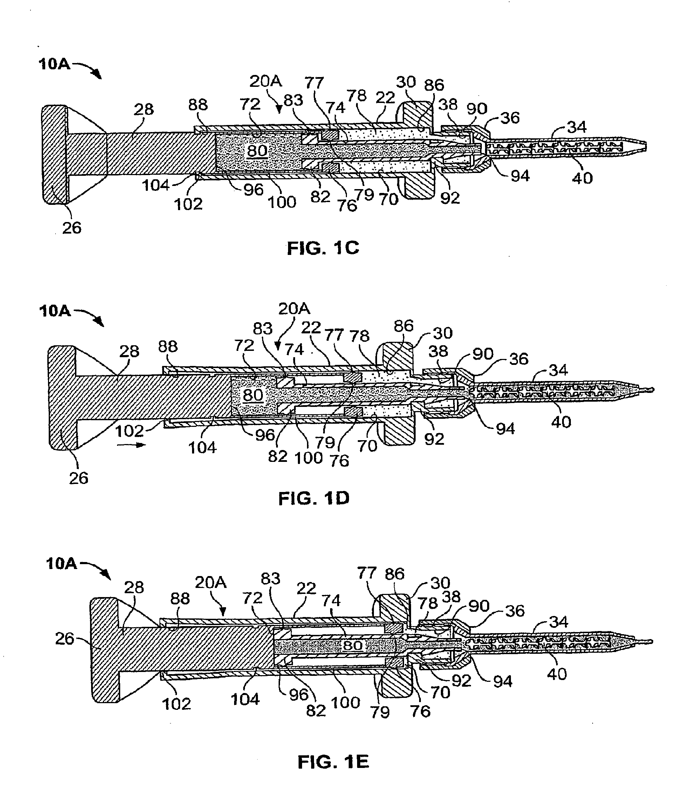

In this embodiment, the dual fluid container 20A includes an outer cartridge wall 22. As explained in detail below, the dual fluid container 20A stores two fluids separate from one another that when mixed together react chemically to form an end product, such as an adhesive. The dual fluid container 20A of this embodiment stores just enough of the component fluids to create a single dose of the end product upon dispensing, which in one embodiment is typically approximately at least 5ml of each component fluid for a total of approximately at least 10ml of fluids in the dual fluid container 20A as a whole. Fig. 1A shows the syringe 10A with a threaded cap 32 in place. The threaded cap 32 is in place during shipping and prior to use. Fig. 1B shows the syringe 10A ready for use with the threaded cap 32 removed and a nozzle 34 attached to the syringe 10A. The nozzle 34 is attached to the syringe 10A by a retaining nut 36, which is threaded onto a threaded outlet 38 of the syringe 10A (Fig. 1C). Typically, the nozzle 34 contains a static mixer 40 within it. The static mixer 40 mixes the two fluids stored in the dual fluid container 20A together as they are dispensed from the syringe 10A.

Referring now to Fig. 1C, a longitudinal sectional view of the syringe embodiment of the single dose dispenser 10 of the present invention is depicted. In this embodiment, the dual fluid container 20A defines a first fluid chamber 70 and a second fluid chamber 72 for storing and dispensing a first fluid 78 and second fluid 80 respectively. In this embodiment of the dispenser 10A, the container 20A includes the outer cartridge wall 22, a delivery tube 74, a first piston 76 having an exterior seal 77 and an interior seal 79 and a compression wall 82 having a seal 83. The seals 77, 79, 83 are annular dimples in this embodiment, but it should be understood that other sealing arrangements may be used (e.g. o-rings). The plunger 28 defines a rear piston surface 96 and includes a transmission structure 100. The plunger 28 in this embodiment does not include a post, but in other embodiments, a post may be used to minimize fluid waste as disclosed in commonly owned U.S. Patent No. 5,310,091 and U.S. Patent Application Ser. No. 11/031,929. The outer cartridge wall 22 in this embodiment is a cylindrical wall defining a hollow interior 86. The outer cartridge wall 22, in this embodiment, at the back end defines an opening 88, which receives the plunger 28. The outer cartridge wall 22 at the other end, the front end, defines a discharge opening 90 and includes the external threaded outlet 38. Further, the outer cartridge wall 22, in this embodiment, has an annular tab 102 formed thereon, and the plunger 28 has an annular notch 104 formed therein. The annular tab 102 seats in the annular notch 104 until the user is ready to use the syringe 10A. This keeps the plunger 28 from advancing prematurely, which would prematurely dispense the fluids 78, 80.

The delivery tube 74 of the container 20A is disposed within the hollow interior 86 of the outer cartridge wall 22, In this embodiment, the delivery tube 74 snaps into locking engagement with the outer cartridge wall 22 at a snap connection 92. The delivery tube 74 defines an outlet 94 that extends within and beyond the outlet 90 in this embodiment. It is foreseen that the outer cartridge wall 22 and the delivery tube 74 may also be formed integral with one another, and it is also foreseen that different outlet configurations for the outlets 90, 94 may be adopted other than the one depicted in the figures. The compression wall 82 in this embodiment is formed integral with the delivery tube 74 which fixes the compression wall 82 in place.

The first piston 76 of the dual fluid container 20A is disposed within the container 20A between the exterior of the delivery tube 74 and the interior of the outer cartridge wall 22. In this embodiment, the first piston 76 surrounds the exterior of the delivery tube 74. The first piston 76, in conjunction with the exterior of the delivery tube 74 and the interior of the outer cartridge wall 22, define the first fluid chamber 70.

In this embodiment, the rear piston surface 96, the transmission structure 100 and the compression wall 82 define the second fluid chamber 72. The delivery tube 74 provides fluid communication between the second fluid chamber 72 and the discharge opening 94. In this embodiment, the transmission structure 100 extends from the rear piston surface 96 of the plunger 28, passes snugly between the compression wall 82 and the interior of the outer cartridge wall 22 forming a seal and is in engagement with the first piston 76.

To dispense the fluids from the syringe 10A, the plunger 28 is pressed forward towards the front of the dispenser 10A. In the embodiment described, this is done by a user grasping the handle 26 and the grip 30 in his hand and squeezing the handle 26 and the grip 30 together. The movement of the handle 26, by the compression of the handle 26 and the grip 30, moves the plunger 28 forward in the dual fluid container 20A, in the direction indicated by the arrow in Fig. 1D. The rear piston surface 96, as a result, pushes against the fluid 80 stored in chamber 72 and the transmission structure 100 presses against the first piston 76. The fluid 80 being pushed by the rear piston surface 96 in the chamber 72 gets compressed by the fixed compression wall 82, pushing the fluid 80 through the delivery tube 74 and through the discharge opening 94, where the fluid 80 is discharged from the dual fluid container 20A. At the same time, the pressing of the transmission structure 100 against the first piston 76 causes the fluid 78 in the first fluid chamber 70 to be pressed into the discharge opening 90, through which the fluid 78 is discharged from the dual fluid container 20A. As the fluids 78, 80 are discharged from the dual fluid container 20A through the discharge openings 90, 94, they are mixed together by the static mixer 40 in the nozzle 34.

This fluid discharge and mixing process continues as long as the plunger 28 is being actuated and as long as fluids are still left to be dispensed from the dual fluid container 20A. Fig. 1D depicts the dual fluid container 20A in an intermediate dispensing position with a portion of the fluids 78, 80 dispensed from the dual fluid container 20A. Fig. 1E depicts the dual fluid container 20A with the fluid contents of the chambers 70, 72 of the container 20A fully dispensed.

The arrangement of the dual fluid container 20A in Fig. 1E, minus the waste fluids 78, 80 shown remaining, is how the dual fluid container 20A looks prior to being filled. To fill the dual fluid container 20A, the chambers 70, 72 are filled with the appropriate fluids 78, 80 through their respective discharge openings 90, 94. The filling process occurs in the reverse manner of the dispensing process described above. During the filling process, air can get trapped in the chambers 70, 72 between the incoming fluids 78, 80 and the piston surfaces 76, 96. Air trapped in the chambers 70, 72 can cause a number of problems in the use of the dual fluid container 20A. Most significantly, air trapped in chambers 70, 72 can negatively impact the ability to control the volumetric dispensing ratio of the fluids 78, 80 in the chambers 70, 72. To alleviate this problem, an air venting system may be employed, such as the air venting system described in commonly owned international patent application number PCT/US03/17997 or United States Patent Application Serial Numbers 10/755,796 and 11/031,929.

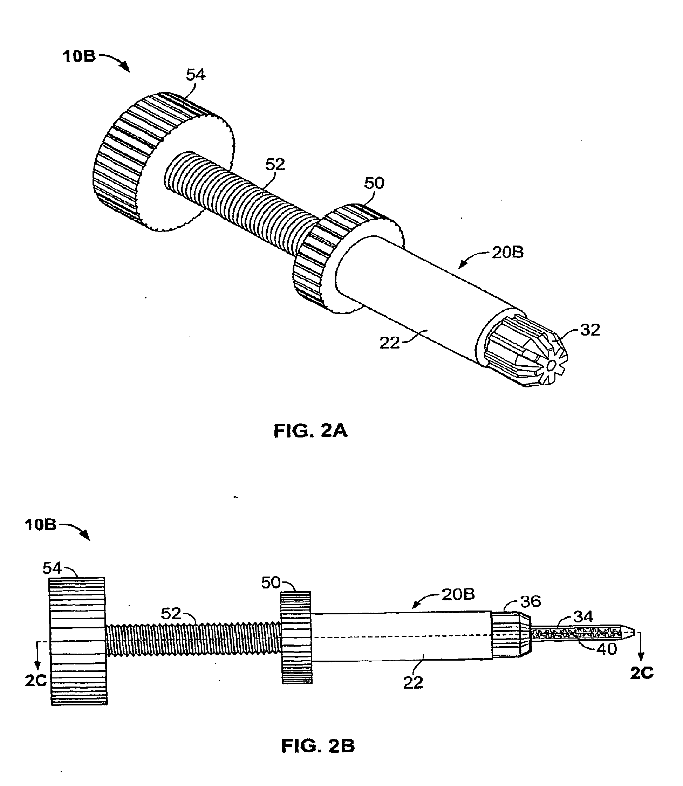

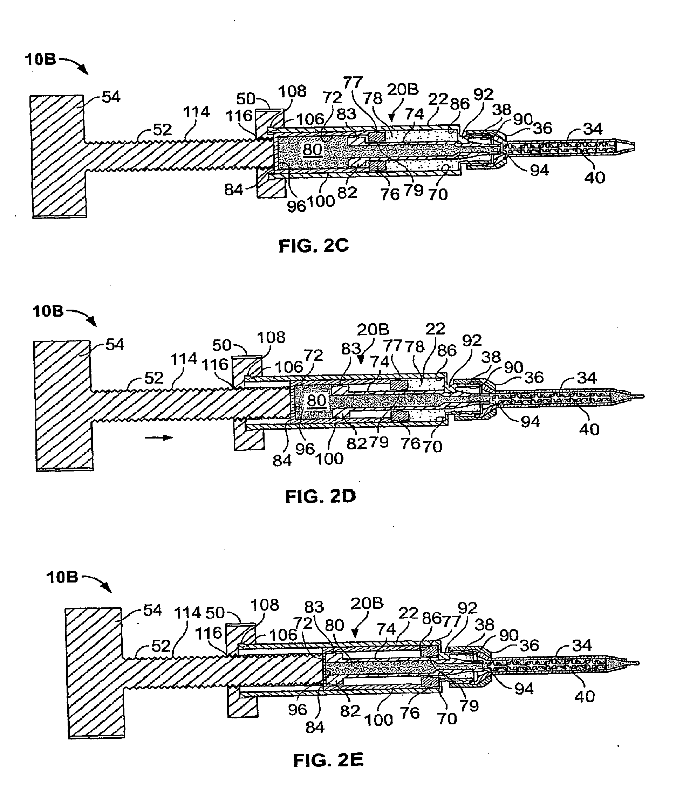

Referring to Figs. 2A-2E, a screw advance mechanism embodiment of the dual fluid dispenser 10B which does not fall within the scope of the present invention is illustrated. In the figures, like elements are numbered the same. The screw dispenser 10B includes an actuation wheel 54, a threaded screw rod 52 having screw threading 114 (Fig. 2C) along the length of the rod 52 and a stationary wheel 50 having counterpart threading 116 formed therein (Fig. 2C). The screw actuated dispenser 10B includes a dual fluid container 20B, similar to the syringe dispenser 10A. Referring to Fig. 2C, in this embodiment, the dual fluid container 20B connects with the stationary wheel 50 through an annular ring 106 formed on the outer cartridge wall 22 that engages a lip 108 formed within the stationary wheel 50. In this embodiment, the dual fluid container 20B has a rear piston assembly 84 which includes the rear piston surface 96 and the transmission structure 100. As with the previous embodiment, the rear piston assembly 84 in this embodiment does not include a post, but in other embodiments, a post may be used to minimize fluid waste as disclosed in commonly owned U.S. Patent No. 5,310,091 and U.S. Patent Application Ser. No. 11/031,929. The rear piston assembly 84 is separate from the screw rod 52 in this embodiment.

To dispense the fluids from the screw mechanism 10B, a user holds the stationary wheel 50 with one hand and rotates the actuation wheel 54 with the other. This causes the screw threading 114 on the screw rod 52 to engage the screw threading 116 formed within the stationary wheel 50, causing the screw rod 52 to advance forward within the dual fluid container 20B, in the direction indicated by the arrow in Fig. 2D. The screw rod 52, as a result, presses against the back of the rear piston assembly 84, causing the rear piston surface 96 to push against the fluid 80 stored in chamber 72 and causing the transmission structure 100 to press against the first piston 76. The fluid 80 being pushed by the rear piston surface 96 in the chamber 72 gets compressed by the fixed compression wall 82, pushing the fluid 80 through the delivery tube 74 and through the discharge opening 94, where the fluid 80 is discharged from the dual fluid container 20B. At the same time, the pressing of the transmission structure 100 against the first piston 76 causes the fluid 78 in the first fluid chamber 70 to be pressed into the discharge opening 90, through which the fluid 78 is discharged from the dual fluid container 20B. As the fluids 78, 80 are discharged from the dual fluid container 20B through the discharge openings 90, 94, they are mixed together by the static mixer 40 in the nozzle 34.

This fluid discharge and mixing process continues as long as the actuation wheel 54 is being turned and as long as fluids are still left to be dispensed from the dual fluid container 20B. Fig. 2D depicts the dual fluid container 20B in an intermediate dispensing position with a portion of the fluids 78, 80 dispensed from the dual fluid container 20B. Fig. 2E depicts the dual fluid container 20B with the fluid contents of the chambers 70, 72 of the container 20B fully dispensed.

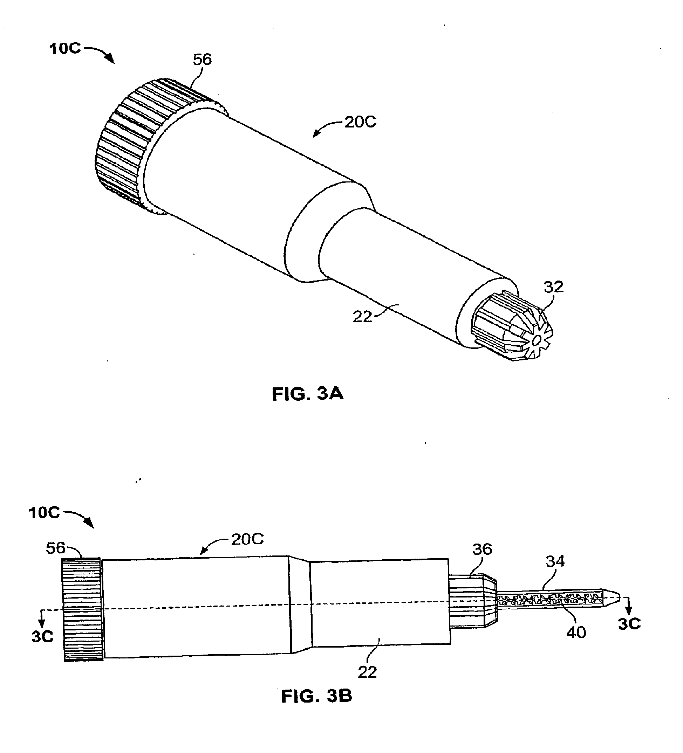

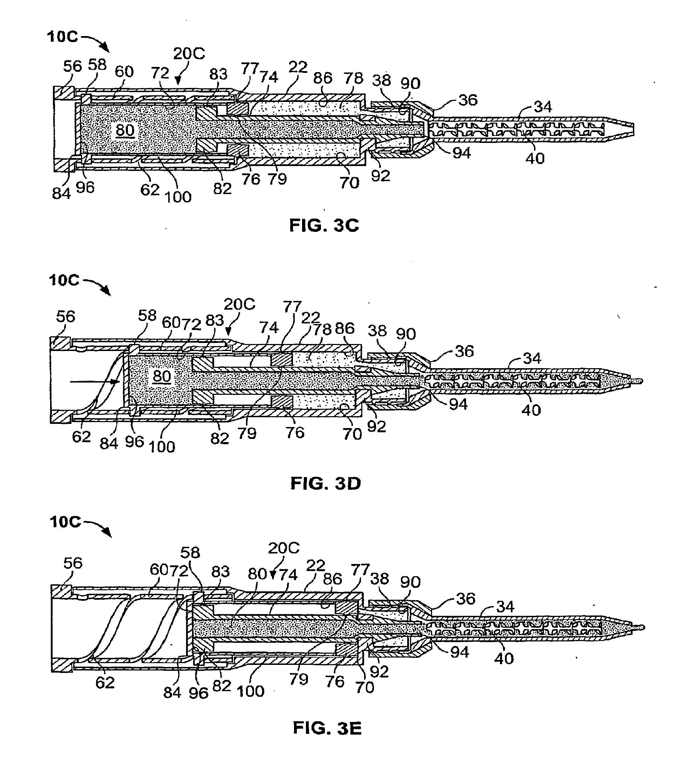

Referring to Figs. 3A-3E, a spiral advance mechanism embodiment of the dual fluid dispenser 10C which does not fall within the scope of the present invention is illustrated. In the figures, like elements are numbered the same. The spiral advance mechanism 10C is similar to a lipstick arrangement. Specifically, referring to Fig. 3C, the spiral advance dispenser 10C includes an actuation wheel 56, a cam wall 60 with a cam groove 62 and a cam follower 58. The spiral advanced dispenser 10C includes a dual fluid container 20C, similar to the other embodiments above. In this embodiment, similar to the screw mechanism 10B, the dual fluid container 20C has a rear piston assembly 84 which includes the rear piston surface 96 and the transmission structure 100. As with the previous embodiments, the rear piston assembly 84 in this embodiment does not include a post, but in other embodiments, a post may be used to minimize fluid waste as disclosed in commonly owned U.S. Patent No. 5,310,091 and U.S. Patent Application Ser. No. 11/031,929. The cam follower 58 is formed integral with the rear piston assembly 84. Further, in this embodiment, the outer cartridge wall 22 of the dual fluid container 20C encloses the cam wall 60.

To dispense the fluids from the spiral advance dispenser 10C, a user holds the outer cartridge wall 22 with one hand and rotates the actuation wheel 56 with the other. This motion rotates the cam wall 60, causing the cam follower 58 to follow the cam groove 62. Since the cam follower 58 is integral with the rear piston assembly 84, the rear piston assembly 84 advances forward within the dual fluid container 20C, in the direction indicated by the arrow in Fig. 3D. The forward movement of the rear piston assembly 84 causes the rear piston surface 96 to push against the fluid 80 stored in chamber 72 and causes the transmission structure 100 to press against the first piston 76. The fluid 80 being pushed by the rear piston surface 96 in the chamber 72 gets compressed by the fixed compression wall 82, pushing the fluid 80 through the delivery tube 74 and through the discharge opening 94, where the fluid 80 is discharged from the dual fluid container 20C. At the same time, the pressing of the transmission structure 100 against the first piston 76 causes the fluid 78 in the first fluid chamber 70 to be pressed into the discharge opening 90, through which the fluid 78 is discharged from the dual fluid container 20C. As the fluids 78, 80 are discharged from the dual fluid container 20C through the discharge openings 90, 94, they are mixed together by the static mixer 40 in the nozzle 34.

This fluid discharge and mixing process continues as long as the actuation wheel 56 is being turned and as long as fluids are still left to be dispensed from the dual fluid container 20C. Fig. 3D depicts the dual fluid container 20C in an intermediate dispensing position with a portion of the fluids 78, 80 dispensed from the dual fluid container 20C. Fig. 3E depicts the dual fluid container 20C with the fluid contents of the chambers 70, 72 of the container 20C fully dispensed.

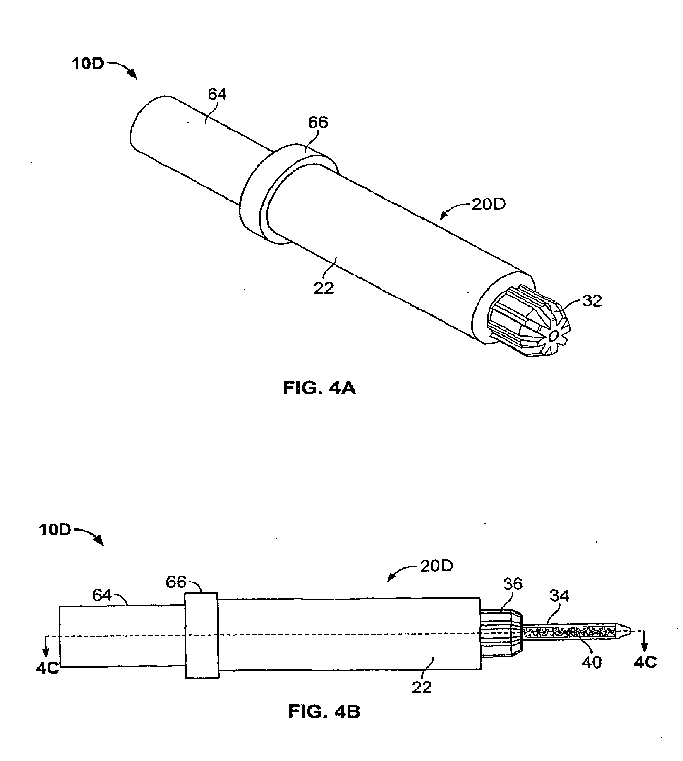

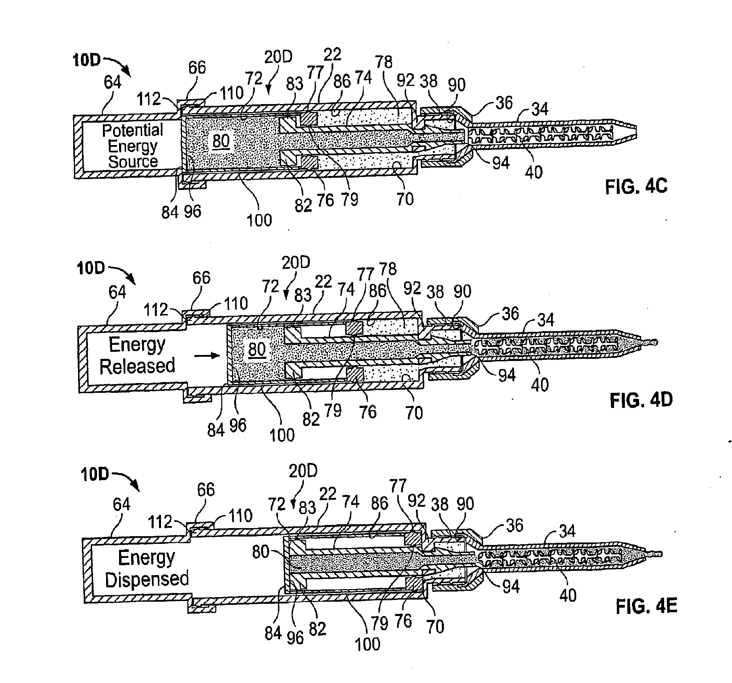

Referring to Figs. 4A-4E a potential energy advance mechanism embodiment of the dual fluid dispenser 10D which does not fall within the scope of the present invention is illustrated. In the figures, like elements are numbered the same. Referring to Fig. 4C, the potential energy dispenser 10D includes a potential energy source, such as, by way of example, a spring or entrapped compressed air, which is contained in container 64. As with the above alternative embodiments, the potential energy actuated dispenser 10D includes a dual fluid container 20D. In this embodiment, the dual fluid container 20D connects with the potential energy container 64 through an annular ring 112 formed on the outer cartridge wall 22 that engages a lip 110 formed in a snap collar 66 of the container 64. As with the other alternative embodiments, the dual fluid container 20D has a rear piston assembly 84 which includes the rear piston surface 96 and the transmission structure 100. As with the previous embodiments, the rear piston assembly 84 in this embodiment does not include a post, but in other embodiments, a post may be used to minimize fluid waste as disclosed in commonly owned U.S. Patent No. 5,310,091 and U.S. Patent Application Ser. No. 11/031,929.

To dispense the fluids from the potential energy dispenser 10D, a user releases and activates the potential energy source, such as by releasing the compressed spring or compressed air, causing the rear piston assembly 84 to advance forward within the dual fluid container 20D, in the direction indicated by the arrow in Fig. 4D. As before, the forward movement of the rear piston assembly 84 causes the rear piston surface 96 to push against the fluid 80 stored in chamber 72 and causes the transmission structure 100 to press against the first piston 76. The fluid 80 being pushed by the rear piston surface 96 in the chamber 72 gets compressed by the fixed compression wall 82, pushing the fluid 80 through the delivery tube 74 and through the discharge opening 94, where the fluid 80 is discharged from the dual fluid container 20D. At the same time, the pressing of the transmission structure 100 against the first piston 76 causes the fluid 78 in the first fluid chamber 70 to be pressed into the discharge opening 90, through which the fluid 78 is discharged from the dual fluid container 20D. As the fluids 78, 80 are discharged from the dual fluid container 20D through the discharge openings 90, 94, they are mixed together by the static mixer 40 in the nozzle 34.

This fluid discharge and mixing process continues until the energy released from the potential energy source is fully dispensed and as long as fluids are still left to be dispensed from the dual fluid container 20D. Fig. 4D depicts the dual fluid container 20D in an intermediate dispensing position with a portion of the fluids 78, 80 dispensed from the dual fluid container 20D. Fig. 4E depicts the dual fluid container 20D with the fluid contents of the chambers 70, 72 of the container 20D fully dispensed.

## Claims

1. A self-contained single dose dual fluid dispenser (10A-10D) to store and dispense two fluids (78, 80), comprising:
a dual fluid container (20A-20D) having an outer cartridge wall (22) having a back end and a front end and defining an outlet (38) at the front end and an open end at the back end, the open end including an annular tab (102);
a delivery tube (74) disposed within the outer cartridge wall (22) and defining an outlet (94) at the front end;
a first piston (76) disposed between the outer cartridge wall (22) and the delivery tube (74) forming a fluid chamber (70) for a single dose of a first fluid (78);
a second piston disposed within the outer cartridge wall (22) between the first piston (76) and the open end of the outer cartridge wall (22), the second piston being longitudinally in-line with the first piston;
a fixed wall (82) disposed between the first piston (76) and the second piston, wherein the fixed wall (82) and the second piston define a second fluid chamber (72) for a single dose of a second fluid (80), the second fluid chamber being longitudinally in-line with the first fluid chamber;
means for providing force to the second piston through the open end of the dual fluid container (20A-20D), wherein the force providing means is attached to the dual fluid container(20A-20D) and includes an annular notch (104) adapted to engage the annular tab (102) of the outer cartridge wall (22) such that said annular tab (102) seats in the annular notch (104) to prevent premature advancement of the second piston; and
means for transmitting force from the second piston to the first piston (76).

2. The self-contained single dose dual fluid dispenser of claim 1, **characterized in that** the:
means for providing force is a force providing mechanism disposed within the open end of and attached to the dual fluid container (20A-20D); and
the means for transmitting force from the second piston is a transmission structure (100) disposed between the second piston and the first piston.

3. The self-contained single dose dual fluid dispenser of claim 2,
wherein the force providing mechanism is a handle (26) with a plunger (28) and a grip (30) assembly.

4. The self-contained single dose dual fluid dispenser of claim 3,
wherein the second piston is formed integral with the plunger (28).

5. The self-contained single dose dual fluid dispenser of any of the above claims,
further comprising a mixer (40) attached to the dual fluid container outlet.

6. The self-contained single dose dual fluid dispenser of any of the above claims,
wherein the delivery tube (74) is formed integral with the fixed wall (82).

7. The self-contained single dose dual fluid dispenser according to any of the above claims,
wherein the force providing mechanism is a potential energy source.

8. A method of dispensing a single dose of a two component end product, comprising:
providing a single dose dual fluid dispenser according to one of claims 1 to 7;
attaching a mixing element to the dual fluid dispenser (10A-10D); and
activating the force providing mechanism, wherein the force providing mechanism applies a force to the second piston of the dual fluid container (20A-20D), which applies a force to the transmission structure (100), which, in turn, presses the first piston (76) and pushes the two fluids (78, 80) contained in the dispenser (10A-10D) out of the mixer and through the attached mixing element to form a single dose of the end product.

9. The method of dispensing a single dose of a two component end product of claim 8,
wherein the force providing mechanism is a handle (26) with a plunger (28) and a grip (30) assembly and wherein the force providing mechanism is activated by grasping the handle (26) and grip (30) and pressing them towards one another, causing the plunger (28) to move within the open end of the dual fluid container (20A-20D) which applies a force to the second piston of the dual container.

10. The method of dispensing a single dose of a two component end product of claim 8,
wherein the force providing mechanism is a screw mechanism (10B) and wherein the force providing mechanism is activated by rotating the screw mechanism (10B), causing the screw mechanism (10B) to apply a force to the second piston of the dual container.

11. The method of dispensing a single dose of a two component end product of claim 8,
wherein the force providing mechanism is a cam mechanism and wherein the force providing mechanism is activated by rotating the cam mechanism, causing the cam mechanism to apply a force to the second piston of the dual container.

12. The method of dispensing a single dose of a two component end product of claim 8, wherein the force providing mechanism is the activation of a potential energy source, which causes the released energy to apply a force to the second piston of the dual container.

## Patentansprüche

1. Eine eigenständige Einzeldosis-Dual-Fluid-Abgabevorrichtung (10A-10D) zum Speichern und Abgeben von zwei Fluiden (78, 80) umfassend:
einen Dual-Fluid-Container (20A-20D) mit einer äußeren Kartuschenwand (22), welche ein hinteres Ende und ein vorderes Ende hat und einen Auslass (38) an dem vorderen Ende und ein offenes Ende an dem hinteren Ende definiert, wobei das offene Ende einen ringförmigen Bund (102) beinhaltet;
ein Zuführrohr (74), welches innerhalb der äußeren Kartuschenwand (22) angeordnet ist und einen Auslass (94) an dem vorderen Ende definiert;
einen ersten Kolben (76), welcher zwischen der äußeren Kartuschenwand (22) und dem Zuführrohr (74) angeordnet ist und eine Fluidkammer (70) für eine Einzeldosis eines ersten Fluides (78) ausgebildet;
einen zweiten Kolben, welcher innerhalb der äußeren Kartuschenwand (22) und zwischen dem ersten Kolben (76) und dem offenen Ende der äußeren Kartuschenwand (22) angeordnet ist, wobei der zweite Kolben in Längsrichtung in Reihe mit dem ersten Kolben angeordnet ist;
eine feste Wand (82), welche zwischen dem ersten Kolben (76) und dem zweiten Kolben angeordnet ist, wobei die feste Wand (82) und der zweite Kolben eine zweite Fluidkammer für eine Einzeldosis eines zweiten Fluides (80) definieren, wobei der zweite Fluidkammer in Längsrichtung in Reihe mit der ersten Fluidkammer angeordnet ist;
Mittel zur Kraftbeaufschlagung des zweiten Kolbens durch das offene Ende des Dual-Fluid-Containers (20A-20D), wobei das Kraftbereitstellungsmittel an dem Dual-Fluid-Container (20A-20D) befestigt ist und eine ringförmige Ausnehmung (104) beinhaltet, welche dazu eingerichtet ist, mit dem ringförmigen Bund (102) der äußeren Kartuschenwand (22) derart in Eingriff zu gelangen, dass der ringförmige Bund (102) in der ringförmigen Nut (104) sitzt, um verfrühtes Vorwärtsbewegen des zweiten Kolbens zu verhindern; und
Mittel zum Übertragen von Kraft von dem zweiten Kolben auf den ersten Kolben (76).

2. Die eigenständige Einzeldosis-Dual-Fluid-Abgabevorrichtung nach Anspruch 1, **dadurch gekennzeichnet, dass** das:
Mittel zur Kraftbeaufschlagung ein Kraftbeaufschlagungsmechanismus ist, welcher an dem Dual-Fluid-Container (20A-20D) befestigt und in dessen offenem Ende angeordnet ist; und
das Mittel zum Übertragen von Kraft von dem zweiten Kolben eine Übertragungsstruktur (100) ist, welche zwischen dem zweiten Kolben und dem ersten Kolben angeordnet ist.

3. Die eigenständige Einzeldosis-Dual-Fluid-Abgabevorrichtung nach Anspruch 2, wobei der Kraftbeaufschlagungsmechanismus ein Handgriff (26) mit einem Plunger (28) und einer Griffanordnung (30) ist.

4. Die eigenständige Einzeldosis-Dual-Fluid-Abgabevorrichtung nach Anspruch 3, wobei der zweite Kolben einstückig mit dem Plunger (28) einstückig ausgebildet ist.

5. Die eigenständige Einzeldosis-Dual-Fluid-Abgabevorrichtung nach einem der obigen Ansprüche,
ferner umfassend einen Mischer (40), welcher an dem Dual-Fluid-Container-Auslass befestigt ist.

6. Die eigenständige Einzeldosis-Dual-Fluid-Abgabevorrichtung nach einem der obigen Ansprüche,
wobei das Zuführrohr (74) einstückig mit der festen Wand (82) einstückig ausgebildet ist.

7. Die eigenständige Einzeldosis-Dual-Fluid-Abgabevorrichtung nach einem der obigen Ansprüche,
wobei der Kraftbeaufschlagungsmechanismus eine Quelle potentieller Energie ist.

8. Ein Verfahren zum Abgeben einer Einzeldosis eines Zweikomponenten-Endprodukts, umfassend:
Bereitstellen einer Einzeldosis-Dual-Fluid-Abgabevorrichtung nach einem der Ansprüche 1 bis 7;
Befestigen eines Misch-Elements an der Dual-Fluid-Abgabevorrichtung (10A-10D); und
Aktivieren des Kraftbeaufschlagungsmechanismus, wobei der Kraftbeaufschlagungsmechanismus eine Kraft auf den zweiten Kolben des Dual-Fluid-Containers (20A-20D) aufbringt, welcher eine Kraft auf die Übertragungsstruktur (100) aufbringt, welche wiederum gegen den ersten Kolben (76) drückt und die zwei Fluide (78, 80), welche in der Abgabevorrichtung (10A-10D) enthalten sind, aus dem Mischer und durch das befestigte Misch-Element drückt, um eine Einzeldosis des Endprodukts auszubilden.

9. Das Verfahren zum Abgeben einer Einzeldosis eines Zweikomponenten-Endprodukts nach Anspruch 8,
wobei der Kraftbeaufschlagungsmechanismus ein Handgriff (26) mit einem Plunger (28) und einer Griffanordnung (30) ist und wobei der Kraftbeaufschlagungsmechanismus durch Ergreifen des Handgriffs (26) und des Griffs (30) und Gegeneinanderdrücken von diesen aktiviert wird, was den Plunger (28) dazu veranlasst, sich innerhalb des offenen Endes des Dual-Fluid-Containers (20A-20D) zu bewegen, was eine Kraft auf den zweiten Kolben des Dual-Containers aufbringt.

10. Das Verfahren zum Abgeben einer Einzeldosis eines Zweikomponenten-Endprodukts nach Anspruch 8,
wobei der Kraftbeaufschlagungsmechanismus ein Schraubmechanismus (10B) ist und wobei der Kraftbeaufschlagungsmechanismus durch Drehen des Schraubmechanismus (10B) aktiviert wird, was den Schraubmechanismus (10B) dazu veranlasst, eine Kraft auf den zweiten Kolben des Dual-Containers aufzubringen.

11. Das Verfahren zum Abgeben einer Einzeldosis eines Zweikomponenten-Endprodukts nach Anspruch 8,
wobei der Kraftbeaufschlagungsmechanismus ein Nockenmechanismus ist und der Kraftbeaufschlagungsmechanismus durch Drehen des Nockenmechanismus aktiviert wird, was den Nockenmechanismus dazu veranlasst, eine Kraft auf den zweiten Kolben des Dual-Containers aufzubringen.

12. Das Verfahren zum Abgeben einer Einzeldosis eines Zweikomponenten-Endprodukts nach Anspruch 8, wobei der Kraftbeaufschlagungsmechanismus die Aktivierung einer Quelle potentieller Energie ist, welche die freigegebene Energie dazu veranlasst, eine Kraft auf den zweiten Kolben des Dual-Containers aufzubringen.

## Revendications

1. Dispositif autonome d'administration de deux fluides à dose unique (10A-10D) pour stocker et administrer deux fluides (78, 80), comprenant :
un récipient à deux fluides (20A-20D) ayant une paroi de cartouche externe (22) ayant une extrémité arrière et une extrémité avant et définissant un orifice de sortie (38) à l'extrémité avant et une extrémité ouverte à l'extrémité arrière, l'extrémité ouverte incluant une languette annulaire (102) ;
un tube de distribution (74) disposé à l'intérieur de la paroi de cartouche externe (22) et définissant un orifice de sortie (94) à l'extrémité avant ;
un premier piston (76) disposé entre la paroi de cartouche externe (22) et le tube de distribution (74) formant une chambre à fluide (70) pour une dose unique d'un premier fluide (78) ;
un second piston disposé à l'intérieur de la paroi de cartouche externe (22) entre le premier piston (76) et l'extrémité ouverte de la paroi de cartouche externe (22), le second piston étant longitudinalement en ligne avec le premier piston ;
une paroi fixe (82) disposée entre le premier piston (76) et le second piston, la paroi fixe (82) et le second piston définissant une seconde chambre à fluide (72) pour une dose unique d'un second fluide (80), la seconde chambre à fluide étant longitudinalement en ligne avec la première chambre à fluide ;
des moyens pour fournir une force au second piston à travers l'extrémité ouverte du récipient à deux fluides (20A-20D), les moyens de fourniture de force étant fixés au récipient à deux fluides (20A-20D) et incluant une encoche annulaire (104) conçue pour se mettre en prise avec la languette annulaire (102) de la paroi de cartouche externe (22) de telle sorte que ladite languette annulaire (102) se positionne dans l'encoche annulaire (104) pour empêcher un avancement prématuré du second piston ; et
des moyens pour transmettre une force du second piston au premier piston (76).

2. Dispositif autonome d'administration de deux fluides à dose unique selon la revendication 1, **caractérisé en ce que** :
les moyens pour fournir une force sont un mécanisme de fourniture de force disposé à l'intérieur de l'extrémité ouverte du récipient à deux fluides (20A-20D) et fixé au récipient à deux fluides ; et
les moyens pour transmettre une force depuis le second piston sont une structure de transmission (100) disposée entre le second piston et le premier piston.

3. Dispositif autonome d'administration de deux fluides à dose unique selon la revendication 2,
**caractérisé en ce que** le mécanisme de fourniture de force est une poignée (26) avec un piston (28) et un ensemble préhenseur (30).

4. Dispositif autonome d'administration de deux fluides à dose unique selon la revendication 3,
**caractérisé en ce que** le second piston est solidaire du piston (28).

5. Dispositif autonome d'administration de deux fluides à dose unique selon l'une quelconque des revendications ci-dessus,
comprenant en outre un mélangeur (40) fixé à l'orifice de sortie du récipient à deux fluides.

6. Dispositif autonome d'administration de deux fluides à dose unique selon l'une quelconque des revendications ci-dessus,
**caractérisé en ce que** le tube de distribution (74) est solidaire de la paroi fixe (82).

7. Dispositif autonome d'administration de deux fluides à dose unique selon l'une quelconque des revendications ci-dessus,
**caractérisé en ce que** le mécanisme de fourniture de force est une source d'énergie potentielle.

8. Procédé d'administration d'une dose unique d'un produit final à deux composants, comprenant :
la fourniture d'un dispositif d'administration de deux fluides à dose unique selon l'une des revendications 1 à 7 ;
la fixation d'un élément de mélange au dispositif d'administration de deux fluides (10A-10D) ; et
l'activation du mécanisme de fourniture de force, le mécanisme de fourniture de force appliquant une force au second piston du récipient à deux fluides (20A-20D), qui applique une force à la structure de transmission (100), qui, à son tour, exerce une pression sur le premier piston (76) et pousse les deux fluides (78, 80) contenus dans le dispositif d'administration (10A-10D) hors du mélangeur et à travers l'élément de mélange fixé pour former une dose unique du produit final.

9. Procédé d'administration d'une dose unique d'un produit final à deux composants selon la revendication 8,
**caractérisé en ce que** le mécanisme de fourniture de force est une poignée (26) dotée d'un piston (28) et d'un ensemble préhenseur (30) et **en ce que** le mécanisme de fourniture de force est activé en saisissant la poignée (26) et le préhenseur (30) et en les pressant l'un vers l'autre, ce qui amène le piston (28) à se déplacer à l'intérieur de l'extrémité ouverte du récipient à deux fluides (20A-20D), appliquant une force sur le second piston du double récipient.

10. Procédé d'administration d'une dose unique d'un produit final à deux composants selon la revendication 8,
**caractérisé en ce que** le mécanisme de fourniture de force est un mécanisme à vis (10B) et **en ce que** le mécanisme de fourniture de force est activé par rotation du mécanisme à vis (10B), ce qui amène le mécanisme à vis (10B) à appliquer une force au second piston du double récipient.

11. Procédé d'administration d'une dose unique d'un produit final à deux composants selon la revendication 8,
**caractérisé en ce que** le mécanisme de fourniture de force est un mécanisme à came et **en ce que** le mécanisme de fourniture de force est activé par rotation du mécanisme à came, ce qui amène le mécanisme à came à appliquer une force au second piston du double récipient.

12. Procédé d'administration d'une dose unique d'un produit final à deux composants selon la revendication 8, **caractérisé en ce que** le mécanisme de fourniture de force est l'activation d'une source d'énergie potentielle, qui amène l'énergie libérée à appliquer une force au second piston du double récipient.
